Europäisches Patentamt

European Patent Office

Office européen des brevets

(19)

(11) Publication number: 0 156 537
A2

(12) EUROPEAN PATENT APPLICATION

(21) Application number: 85301442.1

(22) Date of filing: 01.03.85

(51) Int. Cl.⁴: H 01 F 1/37
H 01 F 1/28

(30) Priority: 02.03.84 US 585888

(43) Date of publication of application:
02.10.85 Bulletin 85/40

(84) Designated Contracting States:
AT BE CH DE FR GB IT LI NL SE

(71) Applicant: THE BOARD OF REGENTS THE UNIVERSITY
OF TEXAS SYSTEM
Texas Medical Center 6 Bartner Avenue
Houston Texas 77030(US)

(72) Inventor: Poynton, Christopher H.
Department of Hemotology Westminster Hospital
Horseferry Road London SW1B 2AP(GB)

(72) Inventor: Reading, Christopher L.
1703 Laurel Spring
Kingwood Texas 77339(US)

(74) Representative: Woodcraft, David Charles
BROOKES & MARTIN High Holborn House 52/54 High
Holborn
London, WC1V 6SE(GB)

(54) Biological magnetic fluids.

(57) A biological magnetic fluid includes a magnetic colloidal disperse phase distributed throughout a liquid dispersion medium. The disperse phase may be made up of magnetic particles coated with cross-linked biologically compatible polymers. The biologically compatible polymers may be connected by way of covalent linkages to biologically active macromolecules. This may be accomplished by covalently linking immunoglobulin to the biologically compatible polymers and then attaching antibodies with predefined specificity to the immunoglobulin. These antibodies may target the magnetic particles on desired cells, for a variety of medical applications. The magnetic particles may be made up of magnetite cores with cobalt or cobalt and boron coatings. In addition magnetic particles may be made of cobalt and boron with the boron being in sufficient concentration to enable radiation activation. The magnetic colloid may be formed by incorporating the biologically compatible polymer into the colloid as the colloid is being formed by reducing a magnetic metallic salt. The colloid is advantageously produced in a multi-stage process in order to achieve very uniform particle sizes. The magnetic colloid may also be made by forming a biologically incompatible magnetic colloid, and slowly adding the colloid to a vigorous biologically compatible polymer. These biological magnetic fluids are useful, for example, in separating cancerous cells from normal cells in bone marrow transplants.

## BIOLOGICAL MAGNETIC FLUIDS

## BACKGROUND OF THE INVENTION

### Field of the Invention

This invention relates generally to magnetic colloidal liquids and particularly to such liquids useful for biological and medical applications.

### Background Art

Metallic colloids began to interest biologists in the 1960's, when their property of binding macromolecules, particularly proteins, almost irreversibly, was discovered. Metallic colloidal gold, the most studied colloidal liquid, is used as a marker, linked to specific carrier proteins for electron microscopy.

A widely described magnetic liquid, known as a ferrofluid, uses magnetite (ferroferric oxide) as the colloidal dispersed phase which is distributed through a liquid dispersion medium such as water, paraffin, toluene or indeed any liquid, including mercury. A surfactant material, or wetting agent, such as oleic acid is usually added to the mixture to prevent the particles from aggregating and ultimately forming a sediment. The surfactant normally binds to the surface of the magnetite particles and keeps them colloidally dispersed in the ferro-fluid.

Dextran coated magnetite particles have also been prepared by researchers. The colloidal magnetite is formed in the concentrated polysaccharide (dextran) solution.    In this way some of the magnetite becomes colloidal, and the rest remains as a sediment.  After heat treatment, some of the dextran bonds to the magnetite particles.   It is known that antibodies can be coupled covalently to the dextran coated magnetite particles by periodate oxidation methods.

The idea of physically and selectively removing unwanted cells from a cell suspension has been studied by a large number of investigators.   Particularly in the treatment of cancer, the removal of cells circumvents numerous   problems   involved   in   using   toxins, chemotherapeutic agents and complement but has been limited to in vitro physical removal.   Density gradient separations have been used to remove lymphocytes from bone marrow cells, but these were not highly efficient.

Various types of magnetic particles have been used for several years in solid phase immunoassays, drug targeting, and more recently, for cell separations.   The magnetic material most often used is magnetite, incorporated into a variety of carrier particles or microspheres with antibody attached.  Very regular polymeric spheres can be made and then coated with other polymers by a gamma irradiation technique to allow adsorption of antibody to the surface.   While cell separations have been attempted using them, these spheres are not easy to prepare and tend to aggregate during protein coupling.   Magnetite-polystyrene spheres have been used for in vitro magnetic cell separations of a neuroblastoma cell line added in various proportions to normal bone marrow. Approximately 97 to 99 percent of the antibody reactive cells could be removed. One problem encountered with magnetic particles of this variety is that they can be pulled off the cell.

The combination of high dose chemotherapy and total body irradiation followed by bone marrow infusion from an identical sibling compatible donor has been developed over the last ten years for the management of malignant hemopoietic disorders. This method has been proven effective in the treatment of relapsed leukemia and has led to a 15 to 20 percent, two year disease free survival rate. The main stumbling block of allogenic bone marrow transplantation is the acute graft-versus-host reaction. One of the main inducing factors of acute graft-versus-host reaction is the infused lymphoid cells, which are present in the bone marrow graft. In spite of donor/recipient major histocompatibility locus (or complex) identity, the frequency of clinical manifestation of donor-versus-host reactions is 70 percent. In approximately 25 percent of the cases, the manifestation is lethal.

The advent of monoclonal antibodies has opened a new frontier for the characterization of cell surface antigens. By immunizing mice, removing the immune spleen cells and hybridizing them with mouse myelomas, many groups have produced cloned antibody producing tumor cells (hybridomas) of predefined specificity. Each of these separate clones produce an antibody to a single determinant of the immunogen. By analysis of the individual specificities of the hybridomas, polyconal antibody response can be segregated into specific and nonspecific clones. In this manner, exquisitely specific immunological reagents can be produced. Since the clones, which remain stable for antibody production and for proliferation, can be grown in culture and as ascites tumors in mice indefinitely, a virtually unlimited supply of these reagents can be prepared, and the identical antibodies can be utilized around the world. The potential for transplantation has been realized by many different groups. Monoclonal antibodies which react with the components on the surface of leukemic cells have been reported by several groups, and some groups have used monoclonal antibody treatment of bone marrow prior to transplantation.

Work has started on techniques for linking a monoclonal antibody to a boron containing compound. Diseased tissue, targeted by the antibody, may be exposed to a "slow" (thermal) neutron beam with the hope that significant cell kill could be obtained. When the isotope of boron $^{10}B$, is exposed to a thermal neutron beam, it decomposes to lithium together with an alpha particle (a helium nucleus), with enough energy to kill cells within a radius of about 10 microns. This technique has been limited because only a few molecules of boron can be attached to a cell with the procedures used.

SUMMARY OF THE INVENTION

In accordance with one preferred embodiment of the present invention, a biological magnetic fluid includes a magnetic colloidal dispersed phase, distributed throughout a liquid dispersion medium. The dispersed phase includes fine magnetic particles coated with cross-linked biologically compatible polymers.

In accordance with another preferred embodiment of the present invention, a biological magnetic fluid includes a magnetic colloidal disperse phase distributed throughout a liquid dispersion medium. The dispersed phase includes magnetic particles containing cobalt and a biologically compatible polymer.

In accordance with still another preferred embodiment of the present invention, a method of making a biological magnetic colloid includes the step of forming a biologically incompatible colloid. The biologically incompatible colloid is then slowly added to a biologically compatible polymer.

In accordance with yet another preferred embodiment of the present invention, a method of making a biological magnetic liquid includes the step of mixing a magnetic metal salt solution with a reducing agent to form a colloid. A low salt biologically compatible polymer is incorporated into the mixture during the formation of the colloid.

## DESCRIPTION OF THE PREFERRED EMBODIMENT

A biological magnetic fluid may include a magnetic colloidal dispersed phase distributed throughout a liquid dispersion medium. The magnetic colloidal dispersed phase may be formed of fine particles of magnetite, magnetite coated with cobalt, cobalt, iron or any other magnetic metal which possesses sufficiently high magnetic properties at the extremely small colloidal particle size to be of use in particular applications. Generally the particles are of a size under 300 nm. for colloid formation, with one preferred particle size being less than 20 nm. Generally, the greater the particle concentration, the more magnetic the liquid. A suitable low salt biologically compatible polymer, that is, a polymer that is compatible with living cells, is incorporated into the colloid. As used herein, "low salt" means having a salt concentration of less than 10 millimolar. The liquid dispersion medium is preferably an aqueous solution such as a physiological salt solution with a pH of about 7.

The incorporation of the low salt biologically compatible polymer into the colloid can be accomplished either in a one step technique, simultaneously with colloid formation or in a two step technique, subsequent to the formation of an initial colloidal liquid. In the two step technique, a conventional biologically incompatible colloid, which may be toxic, is added to a low salt biologically compatible polymer that can withstand rough treatment. Suitable polymers include biologicals such as albumin, yeast mannan, polymerized sucrose, and dextran, or biologically compatible polymers such as polymethacrylate. The rough treatment that must be sustained and that makes a colloid "biologically incompatible", includes exposure to strong oxidizing and reducing conditions and wide pH variations (as wide as from 3 to 11). It is preferred that the biologically incompatible colloid be slowly added to the biologically compatible polymer, not vice versa, and the rate of addition should be slow enough to avoid visually perceptable aggregation. In the one step technique, the colloid is grown directly in the biological material.

The two step technique for incorporating the biologically compatible polymer may be accomplished using a variety of magnetic materials; however, magnetite is advantageous because it is strongly magnetic. A biologically incompatible colloid of magnetite particles or nuclei may be prepared using conventional technqiues, for example, as described in the article by R. Massart, "Preparation of Aqueous Magnetic Liquids in Alkaline and Acidic Media", IEEE Transactions in Magnetism, V.17, No. 2, pp 1247-1248, March 1981, hereby expressly incorporated herein by reference.

Specifically, an aqueous mixture of ferric chloride and ferrous chloride is added to an ammonia solution. The gelatinous precipitate is isolated from the solution by centrifugation or magnetic decantation without washing with water. An alkaline sol may be formed by peptizing the precipitate with a source of highly charged cations, such as aqueous tetramethyl ammonium hydroxide. While an acidic sol is also possible, an alkaline sol is believed to be highly advantageous for most anticipated applications of the present invention. A sol can only be obtained if the Fe (III)/Fe (II) weight ratio is larger than 2, as it is in $Fe_3O_4$. A suitable low salt biologically compatible polymer may be made by dialyzing versus water. The magnetite fluid is slowly added to the relatively vigorous low salt biologically compatible polymer, such as human albumin, until the pH of the albumin is about 9.5. Advantageously, the concentration of the low salt biologically compatible polymer is at least 10 mg./ml. and preferably at least 30 mg./ml. The solution is centrifuged to remove aggregated material after sitting for a sufficient time, and then filtered.

By adding the biologically compatible polymer before the formation of the biological colloid, better binding of the biologically compatible polymer is achieved because greater stabilization of the colloid may be obtained. In fact, it has been found that the biologically compatible polymer often aids in the process of forming the colloid. It may be desirable to form the ultimate colloid in stages. In the first stage, a preliminary colloidal liquid is prepared, and added to biologically compatible polymer. In the second stage, the growth of the colloidal particles is continued, with the biologically compatible polymer contained on the surface of the colloidal particles.

Magnetic colloids may be formed through the chemical reduction of cobalt (II) chloride with sodium borohydride ($NaBH_4$). For cobalt, some of the reaction product is undoubtedly the borides $CO_2B$ and $CoB$, which are also paramagnetic. The same reaction applies to the reduction of iron (II) and (III) chloride, with greater formation of metallic iron rather than iron borides. The formation of the cobalt colloid may be achieved by using either the one step or the two step technique discussed above.

In the one step procedure for making magnetic liquids, it is desirable to first partially reduce the metal salt solution, such as cobalt chloride in a suitable reducing agent such as sodium borohydride and then to repeat the procedure. In fact, it has been found that two applications of the reducing agent and metal salt solution is highly advantageous. More applications may result in ability to vary particle sizes. In addition, it is desirable to regulate or retard the rate of the reduction reaction by incorporating a suitable complexing agent. Suitable agents include the organic acids, such as sodium citrate, sodium succinate and sodium tartrate.

The formation of a colloid with a uniform particle size is important in obtaining a good colloidal suspension. Uniform particle size may be obtained by

using a two step reaction process. In a preferred embodiment the range of particle size is such that the largest particles are less than approximately five times the size of the smallest particles. After a first nucleation reaction, a colloidal metal, such as cobalt, may be grown on the nuclei which may be magnetite particles. It is the number of nuclei first formed that determines the particle size of the colloid, given a finite amount of reactants. Hence, it is possible, by controlled nucleation and growth, to make colloids of a variety of particle sizes.

The metallic cobalt or iron colloid, if left alone, dissolves over a few hours in a solution, so it is usually necessary to passivate the surface. This can be done in a variety of ways, but treatment with a phosphoric acid (in one preferred embodiment approximately 1 to 50 millimolar) and/or potassium dichromate (in one preferred embodiment approximately 1 to 50 millimolar) and/or a zinc salt (in one preferred embodiment approximately 0.1 to 10 millimolar) is effective. The excessive passivating agent is removed by dialysis or ultrafiltration.

It is particularly advantageous to ultrafilter the resulting colloid using tangential flow filtration. The filtration which occurs is extremely effective because the fluid is pumped tangentially across a pressurized filtration membrane so that particles are cleared away from the membrane and blocking is lessened. The purity of the resulting colloid is critical in many applications.

A biologically compatible surfactant may be added to increase the stability of the resulting colloid. Suitable surfactants for biological applications include polyvinyl pyrrolidone and polyethylene glycol. In one preferred embodiment the concentration of the surfactant is from 0.1 mg./ml. to 2 mg./ml.

In the one step procedure for incorporating the biologically compatible polymer into the colloid, colloidal biological magnetite, iron or cobalt may be utilized. The colloidal biological magnetite may be prepared according to the two step technique discussed above and then additional colloid formed, for example, using cobalt. In the one step technique a variety of low salt biologically compatible polymers may be incorporated into the colloid since the colloid is formed with less harsh chemicals. Suitable low salt biologically compatible polymers for incorporation into the colloid with the one step technique include those set forth above for the two step technique as well as naturally occurring polymers such as polysaccharides, glycoproteins, and other proteins, such as lectins. However, it is most economical to first attach a relatively inexpensive, insensitive polymer, such as albumin, as in the two step technique.

It has been discovered that the retention of the biologically compatible polymer on the metallic magnet dispersed phase is greatly improved by cross-linking the biologically compatible polymer to stabilize the surface coating. In effect, a net-like structure of biologically compatible polymer is formed which encircles the metallic particle and is therefore extremely difficult to remove. A variety of cross-linking techniques might be utilized to bind the biologically compatible polymers about the metallic nuclei without causing irreversible aggregation of the colloid. The cross-linking is accomplished for a wide variety of different biologically compatible polymers using glutaraldehyde. In addition, cyanuric chloride and water soluble carbodiimides such as 1-ethyl-3-(3-dimethylaminoproyl) carbodimide (EDCI) may be used for this purpose.

In order to secure a variety of biologically active macromolecules to the individual dispersed phase particles to obtain biologically desirable objectives, the biologically active macromolecules may be adhered via a covalent linkage to the cross-linked biologically compatible polymer wound about the individual metallic nuclei. A variety of methods of forming covalent linkages may be used to accomplish this function including the use of glutaraldehyde, as discussed with respect to the cross-linking step, p-benzoquinone, N-N-dicyclohexylcarbodiimide and diN-hydroxysuccinimidyl succinate. It may sometimes be desirable to use different materials as the biologically compatible polymer adhered to the magnetic particles and as the biologically active macromolecule. For example, one material may be more capable of binding to the magnetic particle and thus more suitable as the biologically compatible polymer, whereas another may have desirable biological activity and thus may be more suitable as the biologically active macromolecule.

The covalent linking operation may be accomplished by first attaching the colloidal particle to the linking agent or by first attaching the biologically active macromolecule to the linking agent and then attaching the colloidal particle. Better results have been obtained by activating the macromolecule with the covalent linking agent and thereafter attaching it to the colloidal particles.

The biologically active macromolecule, such as immunoglobulin, polysaccharides, lectins or other specific proteins, may be activated with a variety of covalent linking agents including p-benzoquinone. The procedure is based on the principle that p-benzoquinone in excess and at near neutral pH reacts with macromolecules by only one of its two active sites. After reaction and removal of excess p-benzoquinone, the activated macromolecule is allowed to react at a slightly alkaline pH, with a suitable secondarily added substance. Benzoquinone is known to react primarily with amino and sulfhydryl groups, but also with other reactive groups present in proteins as well as with polysaccharide moities. The use of p-benzoquinone and other covalent linking agents is described in the article by S. Avrameas, et al., "Coupling of Enzymes to Antibodies and Antigens", in Scand. J. Immunol., Vol. 8, Supp. 7, 7-23, (1978), hereby expressly incorporated by reference herein.

Although not always indispensible, the separation of the biologically active conjugates formed from these derivatives and reagents is often required. Isolated conjugates give less background and have greater specific activity than unpurified preparations. Purification of the conjugates is accomplished by the use of gel filtration columns, depending upon the molecular weight of both the protein and the biologically active macromolecule.

The biological magnetic fluids may be lyophilized for storage and transport and reactivated by adding liquid. In accordance with one preferred embodiment lyophilization is preceded by the addition of at least 10% by volume of fetal bovine serum. The powder may be reconstituted by adding water.

The biological magnetic fluids, made in accordance with the present invention, have many applications. They may be used for cell separations, to target drugs, as a "target" for radiation treatment, or to block arteries. It may sometimes be advantageous to incorporate radioactive markers in the magnetic liquid. This may be done using isotopes of iron or cobalt such as cobalt 57.

The biologically active macromolecules attached to the individual colloidal magnetic particles may be bound to monoclonal or polyclonal antibodies which attack and adhere to specific cells of biological interest, such as cancer cells. Because of the magnetic properties of the colloid, cell separations may be accomplished. For example, cancerous bone marrow cells may be magnetically separated from normal cells.

The high boride containing biological magnetic liquid made by reducing a metal salt solution with a reducing agent can be used to coat selected cell populations from a mixture of cells in vitro (such as cancer cells in human bone marrow). The cancer or other cells can be selected by an appropriately specific monoclonal antibody adhered on the colloidal particles, as described above. After careful washing of the cells to remove excess (free) colloid, the cells can be dispersed evenly in a suitable medium, such as gelatin (approx. 1% concentration), to prevent sedimentation of the cells. The cells can then be exposed to a thermal neutron beam, causing the boron atoms to emit a high energy alpha particle, thereby killing the colloid coated cells, while leaving the others viable and undamaged. In this way bone marrow can be purged of cancer cells or other cells. Because of the high density of dispersed boron atoms obtainable with the present invention, the probability of achieving a boron/neutron collision would be greatly increased. Specifically, in one preferred embodiment each colloidal particle would be at least about 5% boron which means there would be in the neighborhood of 100,000 $^{10}$B

molecules per colloid particle and $5 \times 10^9$ $^{10}B$ molecules per cell (50,000 colloid particles estimated per cell).

The technology for making erythrocyte ghosts is well established (e.g. Schwoch & Passow, Molecular & Cellular Biochemistry, Vol. 2, No 2, pp. 197-218, December 15, 1973, hereby expressly incorporated herein by reference). Human albumin magnetic fluid can be used as a filler for erythrocyte ghosts, immediately after formation of the fluid, without covalently linking other functional molecules. This fluid and any additions to it, such as drugs, or radioactive markers can be incorporated into resealed erythrocyte ghosts by mixing a suspension of erythrocytes with the concentrated magnetic fluid.

The mixture is then diluted with a hypotonic magnesium/TRIS ion containing buffer to lyse the erythrocytes, which are then resealed after a few minutes by restoring the osmolarity to physiological levels with, for example, sodium chloride. The cells thus encapsulate the magnetic fluid and its added substances, and can be separated and purified by a variety of methods such as density gradient centrifugation.

These magnetic erythrocyte ghosts can be used to deliver substances to medically or biologically desirable parts of the body by magnetic methods. There may be advantages to using autologous ghosts, since they will not be recognized as foreign, at least initially, by the body.

The invention may be better understood through the consideration of the following examples:

## Example 1

Preparation of a Magnetite Cobalt Magnetic Colloid

(a) Formation of Colloidal Magnetite in Protein Solution

A magnetite fluid (12 nm. magnetite particles), made with an aqueous mixture of ferric chloride (40 ml., 1 molar) and ferrous chloride (10 ml. 2 molar, in HCl 2 molar), is added to ammonia solution (500 ml. 0.7 molar) at room temperature. The gelatinous precipitate is isolated from solution by centrifugation or magnetic decantation without washing with water. An alkaline ferrofluid is made by peptizing the precipitate with aqueous tetramethyl ammonium hydroxide, which is added until its concentration in the mix is at least one molar or about 180 mg./ml. The sol can be obtained only if the Fe (III) to Fe (ll) weight ratio is greater than two.

The magnetic fluid is slowly added to the low salt biologically compatible polymer (50 ml. of 30 mg./ml. (3%) human albumin, obtained from Baxter Travenol, Deerfield, Illinois) until the pH of the albumin is about 9.5, which is optimal for reduction by sodium borohydride. A deep brown solution with minimal aggregation results and should be left overnight at 4°C.

The solution is centrifuged at about 1000 g's for 20 minutes and filtered through a low protein binding, 0.22 micron filter. The volume is adjusted to 50 milliliters with water and the resulting material is stored at $4^{O}C$.

(b) The Growth of Cobalt/Boride on Particles and Passivation

One molar sodium borohydride ($NaBH_4$) (300 microliters) is mixed with the 50 ml. of solution produced in part (a) above at room temperature. A freshly prepared cobalt chloride solution (150 microliters) (0.5 molar $CoCl_2$ and 0.5 molar sodium citrate, in the volume ratio of 3:2) is added and quickly mixed. The material is left for 5 minutes and then one molar sodium borohydride (1 ml.) is added with mixing.

Additional cobalt chloride/sodium citrate solution (2 ml.) is added and mixed as above, but this time it is allowed to sit for 15 minutes.

Potassium dichromate is added until the final concentration is 15 millimolar and zinc chloride is added to 5 millimolar, to prevent oxidization. Polyvinyl pyrrolidone (PVP) (MW 40,000) (to one milligram per milliliter) is added as a biologically safe surfactant which increases the stability of the colloid.

The colloid is ultrafiltered using a tangential flow AMICON with a 30 nanometer membrane, to obtain about 15 milliliters. The colloid is then filtered through an 0.22 micron filter, and run in 10 milliliter aliquots on a gel filtration column. Suitable gel filtration beads include Sepharose CL 6B (250 milliliters), and a suitable column is 20 centimeters by 3.5 centimeters. The gel is equilibrated with PVP (one milligram per milliliter in 10 millimolars of $NaHCO_3$.

(c) Cross-linking of Albumin to Stabilize Surface Coating

The pH of the colloid is adjusted to 6.8 with phosphate buffer (to 50 millimolar final concentration in a volume of 200 milliliters). Next, 1.5% glutaraldehyde is added and the material is allowed to sit at room temperature for eighteen hours. Thereafter, ultrafiltration is carried out to 10-15 milliliters using a 50 nm. membrane.

(d) Forming the Covalent Linkage for Macromolecules

Immunoglobulin (affinity purified fraction of goat anti-mouse immunoglobulins, obtained from Boehringer Mannheim Biochemicals, Indianapolis, Indiana) is activated with p-benzoquinone. To one milligram of immunoglobulin and 0.4 milliliters of 0.1 molar phosphate buffer (pH 6.0) add 0.1 milliliter p-benzoquinone and 95% ethanol (30 milligrams/milliliters). The liquid is kept in the dark for an hour. Thereafter, it is filtered through a fine column (Sephadex 0-25, 0.9 centimeters by 4 centimeters) equilibrated with 0.15 molar NaCl. The first colored fraction containing the activated protein is collected in a volume of approximately 1 milliliter. One-tenth of the volume of 1 molar $NaHCO_3$ is added to the benzoquinone activated immunoglobulin and the mixture is added to the concentrated colloid in 0.1 molar $NaHCO_3/Na_2CO_3$ (pH 9.0) (1 milligram protein/5 milliliters colloid). The liquid is left for 48 hours at $4^O$C. It is then run on a gel filtration column (Sepharose CL 6B equilibrated with phosphate buffered saline, pH 7.4) and sterile filtered using a 0.22 micron low protein binding filter.

## Example 2

Alternate Formation of a Colloidal Magnetite/Cobalt Liquid

The procedure outlined in paragraphs (a)-(c) of Example 1 is followed. However, the covalent linkage may be formed by the activation of the colloid with p-benzoquinone. This is done by running the colloid (10 milliliter aliquots) on a gel filtration column (Sepharose, 250 milliliters CL 6B) equilibrated with PVP, 1 milligram per milliliter, in a 10 millimolar phosphate buffer at pH 8.0. The pH is then adjusted to 6 with phosphate buffer to a final concentration of 50 millimolar. A one quarter volume of p-benzoquinone (30 mg./ml.) in 95% ethanol is added and the mixture is kept in the dark for an hour with occasional mixing. The product is run on a column equilibrated with 10 millimolar $NaHCO_3$, and then concentrated by ultrafiltration to about 10 milliliters. The pH is adjusted to 9, so that the colloid is in 0.1 molar $NaHCO_3/Na_2CO_3$ buffer. The immunoglobulin is added to a concentration of 1 milligram per 5 milliliters of concentrated colloid. The product is left for 48 hours at 4°C to bind. Thereafter, it is run on a gel filtration column, equilibrated with phosphate buffered saline, pH 7.4.

Example 3

Formation of a High Boride Containing Magnetic Fluid Low salt human albumin (50 ml., 30 mg./ml.) is mixed with one molar sodium borohydride (300 microliters). Then 150 microliters of freshly prepared cobalt solution (0.5 molar cobalt chloride and 0.5 molar sodium citrate in a volume ratio of 3:2) is mixed in quickly. After the liquid is allowed to sit for five minutes, one molar sodium borohydride (1 ml.) is mixed in. Then 2 milliliters of the cobalt chloride/sodium citrate mixture are added and mixed.

After the liquid has been allowed to sit for 15 minutes, potassium dichromate and zinc chloride and polyethylene glycol (20,000 MW) may be added as discussed in Example 1. Ultrafiltration and gel filtration may be accomplished also as described with respect to Example 1.

## Example 4

Separation of Various Bone Marrow Cells

The colloid produced in accordance with Examples 1, 2 or 3 may be used to separate bone marrow cells into cancerous and noncancerous components or to remove graft versus host disease causing cells. This may be done by collecting bone marrow cells (1,000 cc.) from a donor by multiple aspirations from the iliac crest under general anesthesia. The cells should be kept at less than $10^{\circ}$C. Cells are passed through a hemonetic cell separator or gradient centrifuge to remove granulocytes and red cells. To remove graft versus host disease causing cells, the cells are incubated with mouse monoclonal antibody (CT-2) for 45 minutes at $4^{\circ}$C which reacts with E-rosette receptor positive lymphocytes (T-cells). The CT-2 antibody, a pan T-cell monoclonal antibody, is described in the article by M. Trigg, R. Billing et al., "In Vitro Treatment of Donor Bone Marrow with Anti E-rosette Antibody and Complement Prior to Transplantation", J. Cell Biochem. 7A(Supplement):57, 1983, hereby expressly incorporated by reference herein. Three washes by centrifugation of the product are undertaken with Hepes buffered Hanks balanced salt solution (HHBS) at pH 7.4 with 2% by volume fetal bovine serum (FBS).

After resuspension, the cells are incubated with affinity purified immunoglobulin fraction goat antimouse antibody bound to the magnetic fluid, made in accordance with Example 1 with 10% by volume fetal bovine albumin, for 60 minutes at 4°C. Magnetic separation is undertaken with a permanent magnet for 60 minutes at 4°C to remove antibody colloid coated cells. Alternatively, a flow through magnetic chamber may be used with HHBS at pH 7.4 and 2% by volume FBS. The chamber may be made of a stack of U-shaped cobalt/samarium permanent magnets, developing 8,500 gauss, with concave pole faces and interior grooving of the individual magnets to focus the magnetic gradients at the passing cells. A coil or mesh of magnetic wire is contained within a siliconized glass tube held within the gap formed by the U-shaped magnets. The cells flow through the chamber by way of the siliconized tube.

The remaining cells are removed by decanting. The material is washed with Hepes buffered Hanks balanced salt solution, and filtered using a glass filter with a pore size of 40 to 80 microns followed with washing in Hanks balanced salt solution. The liquid is sampled for in vitro progenitor testing and measurement of separation efficiency. Cells are collected for intravenous infusion over thirty minutes.

Monoclonal antibody-reactive cells that escape the separation procedure are detected in a variety of ways. Immunofluorescence may be used either with a second antibody such as goat antimouse immunoglobulin, labeled with fluororescein or with a third antibody reactive with the colloid-bound antibody (rabbit antigoat immunoglobulin) labeled with fluorescein. In addition, the leukemic cells are radioactively prelabeled in culture prior to separation in mixtures with unlabeled normal bone marrow cells. Also clonogenic leukemic cells are grown in a culture after separation. Finally, a biological model using animal leukemia (rat or mouse) is used whereby even one viable remaining leukemic cell can be detected.

Antibody reactive cells have been removed to the limit of immunofluorescence (less than 1% remaining) with the recovery of antibody negative cells above 50 percent.

While the present invention has been described with respect to a limited number of embodiments, those skilled in the art will appreciate a number of modifications and variations. It is intended within the appended claims to cover all such modifications and variations as come within the true spirit and scope of the claims.

FEATURES OF THE INVENTION ARE SUMMARISED AS FOLLOWS:

1.     A biological magnetic liquid comprising a magnetic colloidal disperse phase distributed through a liquid dispersion medium, said disperse phase including fine magnetic particles coated with a cross-linked biologically compatible polymer.

2.     The liquid of claim 1, wherein said magnetic particles include magnetite as a substantial component.

3.     The liquid of claim 1, wherein said particles include cobalt as a substantial component.

4.     The liquid of claim 1, wherein said particles include boron.

5.     The liquid of claim 1, wherein said magnetic particles include cobalt and boron.

6.     The liquid of claim 1, wherein said biologically compatible polymer is a biological polymer.

7.     The liquid of claim 6, wherein said polymer is human albumin.

8.     The liquid of claim 1, wherein said liquid dispersion

26

0156537

medium is an aqueous solution.

9.   The liquid of claim 8 wherein said liquid dispersion medium is formed substantially of a physiological salts.

10.   The liquid of claim 1, including a further surfactant.

11.   The liquid of claim 10, wherein said surfactant is polyvinyl pyrrolidone.

12.   The liquid of claim 10, wherein said surfactant is polyethylene glycol.

13.   The liquid of claim 1, including a covalent linking agent connected to said cross-linked biologically compatible polymer.

14.   The liquid of claim 13, wherein antibodies are connected via said covalent linking agent.

15.   The liquid of claim 13, wherein said covalent linking agent is p-benzoquinone.

16.   The liquid of claim 13, wherein a biologically active macromolecule is attached via said covalent linking agent to said biologically compatible polymer coated magnetic particles.

17. A biological magnetic liquid comprising a magnetic colloidal disperse phase distributed throughout a liquid dispersion medium, said disperse phase including magnetic particles comprising biologically compatible polymer and cobalt and optionally another magnetic metal.

18. The liquid of claim 17 wherein said biologically compatible polymer is human albumin.

19. The liquid of claim 17 wherein said liquid dispersion medium is an aqueous solution.

20. The liquid of claim 17, wherein said magnetic particles are composed of a substantial component of magnetite.

21. The liquid of claim 17, wherein said magnetic particles contain boron.

22. The liquid of claim 21, wherein said magnetic particles contain at least about 5% boron.

23. A method of making a biological magnetic liquid comprising the steps of:

    (a) forming a biologically incompatible colloid; and

    (b) slowly adding the colloid to a liquid, low salt biological polymer, such as to avoid substantial aggregation to form a biologically compatible colloid.

24.   The method of claim 23, including the step of subjecting the liquid to tangential flow ultrafiltration.

25.   The method of claim 23, including the step of cross-linking said biologically compatible polymer.

26.   The method of claim 23, including the step of forming a covalent linkage on said biologically compatible polymer to secure a colloidal particle to a biologically active macromolecule.

27.   The method of claim 26, including the step of connecting an antibody to said covalent linkage by way of immunoglobulin.

28.   The method of claim 27, wherein said immunoglubulin is activated with a suitable linkage and the activated immunoglobulin with the linkage attached is then connected to a magnetic colloidal particle.

29.   The method of claim 23 including the steps of adding a reducing agent and a magnetic metal salt solution to said biological colloid.

30. The method of claim 23 including the steps of adding fetal bovine serum and then lyophilizing the magnetic liquid.

31. A method of making a biological magnetic liquid comprising the step of mixing a magnetic metal salt solution with a reducing agent to form a colloid in the presence of a low salt biologically compatible polymer to form the colloid.

32. The method of claim 31, wherein said magnetic metal salt solution includes cobalt as a substantial component.

33. The method of claim 31, wherein said reducing agent includes boron.

34. The method of claim 31 wherein said biologically compatible polymer is a biological polymer.

35. The method of claim 31 including the step of adding a complexing agent to slow the formation of the colloid.

36. The method of claim 31 including the step of passivating the particle surfaces of the colloid.

37. The method of claim 31 including the step of adding a further biological surfactant.

38. The method of claim 31 including the step of ultrafiltering the colloid.

41. The method of claim 31 including the step of cross-linking said biologically compatible polymer.

42. The method of claim 31 including the step of forming a covalent linkage on said biologically compatible polymer.

43. The method of claim 42 including the step of connecting an antibody to said covalent linkage by way of immunoglobulin.

44. The method of claim 43 wherein said immunoglobulin is activated with a covalent linkage and then the activated immunoglobulin with the linkage attached is connected to a magnetic colloidal particle.

45. The method of claim 42 wherein said covalent linkage attached to a biologically active macromolecule, is attached to said biologically compatible polymer.

46. The method of claim 31, wherein said liquid is lyophilized in a mixture with at least 10% by volume fetal bovine serum.

47. A lyophilized biological magnetic colloid comprising magnetic colloidal particles coated with a cross-linked biologically compatible polymer.

48. The colloid of claim 47 including lyophilized fetal bovine serum.

49. A lyophilized biological magnetic colloid comprising magnetic colloidal particles coated with cobalt and biologically compatible polymer.

50. The colloid of claim 49 including lyophilized fetal bovine serum.

CLAIMS

1.    A biological magnetic liquid comprising a magnetic colloidal disperse phase distributed through a liquid dispersion medium, said disperse phase including fine magnetic particles coated with a cross-linked biologically compatible polymer.

2.    A liquid according to claim 1, wherein said magnetic particles include magnetite cobalt and/or boron as a substantial component.

3.    A liquid according to claim 1, wherein said biologically compatible polymer is a biological polymer.

4.    A liquid according to claim 1, wherein said liquid dispersion medium is an aqueous solution formed substantially of physiological salts.

5.    A liquid according to claim 1, including a covalent linking agent connected to said cross-linked biologically compatible polymer.

6.    A liquid according to claim 1, wherein antibodies are connected via said covalent linking agent.

7.    A biological magnetic liquid comprising a magnetic colloidal disperse phase distributed throughout a liquid dispersion medium, said disperse phase including magnetic particles comprising biologically compatible polymer and cobalt and optionally another magnetic metal.

8.    A method of making a biological magnetic liquid comprising the steps of:

   (a)  forming a biologically incompatible colloid,

and

(b)  slowly adding the colloid to a liquid, low salt biological polymer, such as to avoid substantial aggregation to form a biologically compatible colloid.

9.  A method according to claim 8 including the step of subjecting the liquid to tangential flow ultrafiltration.

10.  A method according to claim 8, including the step of forming a covalent linkage on said biologically compatible polymer to secure a colloidal particle to a biologically active macromolecule.

11.  A method according to claim 10, including the step of connecting an antibody to said covalent linkage by way of immunoglobulin.

12.  A method according to any one of claims 8 to 11 which includes the steps of adding fetal bovine serum and then lyophilizing the magnetic liquid.

13.  A lyophilized biological magnetic colloid comprising magnetic colloidal particles coated with a cross-linked biologically compatible polymer.

14.  A colloid according to claim 13 including lyophilized fetal bovine serum.

15.  A colloid according to claim 13 or 14, wherein the particles are coated with cobalt and a biologically compatible polymer.